# EUROPÄISCHE PATENTSCHRIFT

(11) **EP 1 439 789 B1**
(45) Veröffentlichungstag und Bekanntmachung des Hinweises auf die Patenterteilung: **26.05.2010**
(21) Anmeldenummer: 02754947.6
(22) Anmeldetag: 26.07.2002
(51) Int. Cl.: A61B 17/70

(54) **KNOCHENFIXATIONSEINRICHTUNG UND SCHRAUBE FÜR EINE SOLCHE**
BONE FIXATION DEVICE AND SCREW THEREFOR
DISPOSITIF DE FIXATION OSSEUSE ET VIS ASSOCIEE

(30) Priorität: 23.10.2001 EP 01125150
(43) Veröffentlichungstag der Anmeldung: 28.07.2004
(73) Patentinhaber: BIEDERMANN MOTECH GmbH, 78054 VS-Schwenningen (DE)
(72) Erfinder: BIEDERMANN, Lutz, 78048 VS-Villingen (DE); OSTERMANN, Peter, 46397 Bocholt (DE); HARMS, Jürgen, 76227 Karlsruhe (DE)
(74) Vertreter: Hofer, Dorothea
(86) Internationale Anmeldenummer: PCT/EP2002/008358
(87) Internationale Veröffentlichungsnummer: WO 2003/034930

(56) Entgegenhaltungen:
- FR-A- 2 702 361
- FR-A- 2 727 620
- US-A- 5 002 542
- US-A- 5 024 213
- US-A- 5 584 831
- US-B1- 6 179 838

## Beschreibung

Die Erfindung betrifft eine Einrichtung zur Fixation von Knochen, insbesondere am Becken oder von Röhrenknochen sowie Schrauben für solch eine Einrichtung.

Zur Fixation von Knochen, insbesondere am Becken oder von Röhrenknochen bei einer Fraktur werden in bekannter Weise Platten und Schrauben verwendet. Diese sind so ausgebildet, dass sie der Wölbung des zu fixierenden Knochens bzw. Beckenteiles einigermaßen anpassbar sind. Die Plattenform selbst und die Stellung der Schrauben hierzu sind nur in geringem Maß veränderbar.

Aus der US 5,002,542 ist eine Klemme für eine Knochenschraube bekannt, die zwei Abschnitte aufweist zur Aufnahme des Kopfes der Knochenschraube, einen Haken, der einen Stab aufnimmt und ein Klemmmittel, das die beiden Abschnitte zusammenhält, so dass sie den Kopf der Knochenschraube und den von dem Haken geführten Stab festklemmen. Durch die große Anzahl verschiedener Einzelteile ist diese Klemme jedoch sehr kompliziert zu handhaben und aufwändig in ihrer Herstellung.

Die US 5,584,831 zeigt eine Knochenfixationseinrichtung mit einer Knochenschraube und einer Klemme. Die Klemme besteht aus zwei Teilen, die auf der einen Seite kugelsegmentförmige Ausnehmungen zur Aufnahme des kugelförmigen Kopfs der Knochenschraube und auf der anderen Seite zylindersegmentförmige Ausnehmungen zur Aufnahme eines Stabes aufweisen. Die beiden Teile werden über eine Schraube so miteinander verbunden, dass sie einerseits den Kopf der Knochenschraube und andererseits den Stab festklemmen. Auch diese Fixationseinrichtung ist kompliziert zu handhaben und aufwändig in ihrer Herstellung.

Aufgabe der Erfindung ist es, eine Fixationseinrichtung und Schrauben für eine solche zu schaffen, mit denen eine Repositionierung und Fixation, z.B. eines Beckens oder von Röhrenknochen, wesentlich verbessert werden kann und insbesondere eine Fixationseinrichtung mit geringer Bauhöhe bereitzustellen. Weiterhin soll die Erfindung eine Fixationseinrichtung bereitstellen, die möglichst kostengünstig zu realisieren und möglichst einfach zu handhaben ist. Weiterhin soll die Erfindung eine Fixationseinrichtung bereitstellen, bei der die Fassung mit der Schraube gemeinsam eingesetzt werden kann. Weiterhin soll die Erfindung eine Fixationseinrichtung bereitstellen, die in der Lage ist, mehr als einen Stab zu fixieren.

Diese Aufgabe wird durch die in Patentanspruch 1 gekennzeichnete Einrichtung bzw. durch die in Patentanspruch 2 gekennzeichnete Fixationseinrichtung gelöst. Weiterbildungen der Erfindung sind in den Unteransprüchen gekennzeichnet.

Eine Fixationseinrichtung weist zumindest eine Schraube auf mit einem Schraubenelement und einer das Schraubenelement und einen Stab fixierenden Fassung, die ein Oberteil und ein Unterteil aufweist. Das Schraubenelement weist ein Gewindeschaftteil mit Gewinde und einen kugelförmigen Kopf auf. Oberteil und Unterteil der Fassung weisen auf der einen Seite kugelsegmentförmige Ausnehmungen zur Aufnahme des kugelförmigen Kopfs der Knochenschraube und auf der anderen Seite zylindersegmentförmige Ausnehmungen zur Aufnahme eines Stabes auf. Sie werden durch eine mit einem zwischen der kugelsegmentförmigen und der zylindersegmentförmigen Ausnehmung ausgebildeten Gewinde zusammenwirkenden Schraube miteinander verbunden, wodurch gleichzeitig der kugelförmige Kopf der Schraube und der Stab fixiert werden.

Vorzugsweise sind Oberteil und Unterteil als flache plattenförmige Teile mit identischem Aufbau ausgeführt und werden symmetrisch zu einer durch die Mittellinie des Stabs und den Mittelpunkt des kugelförmigen Kopfs der Schraube definierten Ebene angeordnet. Dadurch kann eine geringe Bauhöhe erzielt werden. Außerdem ist dadurch die Anzahl bereitzustellender Teile verringert, wodurch die Fixationseinrichtung kostengünstiger herzustellen und einfacher zu handhaben ist.

Bei einer identischen Ausführung von Oberteil und Unterteil entspricht der Ausnehmung zum Durchführen des Gewindeschaftteils im Unterteil eine Ausnehmung zum Durchführen eines Werkzeugs zum Drehen des Schraubelements im Oberteil. Dadurch kann die Schraube mit vormontierter Fassung in den Knochen eingeschraubt werden, was die Handhabung vereinfacht.

Vorzugsweise sind Oberteil und Unterteil der Fassung so ausgebildet, dass jeweils in der Mitte eine kugelsegmentförmige Ausnehmung zur Aufnahme des kugelförmigen Kopfs ausgebildet ist, zu beiden Seiten je ein Gewinde zum Verbinden von Oberteil und Unterteil ausgebildet ist und an den beiden Enden je eine zylindersegmentförmige Ausnehmung zur Aufnahme je eines Stabes ausgebildet ist. Auf diese Weise ist es möglich, mehr als einen Stab mit einer Schraube zu verbinden.

Weitere Merkmale und Zweckmäßigkeiten der Erfindung ergeben sich aus der Beschreibung von Ausführungsbeispielen anhand der Figuren. Von den Figuren zeigen:
- Fig. 1: eine seitliche Schnittdarstellung einer ersten Ausführungsform;
- Fig. 2: eine perspektivische Darstellung einer zweiten Ausführungsform;
- Fig. 3: eine seitliche Schnittdarstellung entlang Linie III-III in Fig. 2;
- Fig. 4: eine perspektivische Ansicht einer dritten Ausführungsform;
- Fig. 5: eine seitliche Schnittdarstellung entlang Linie III-III in Fig. 2 für eine vierte Ausführungsform;
- Fig. 6: eine perspektivische Ansicht einer fünften Ausführungsform.

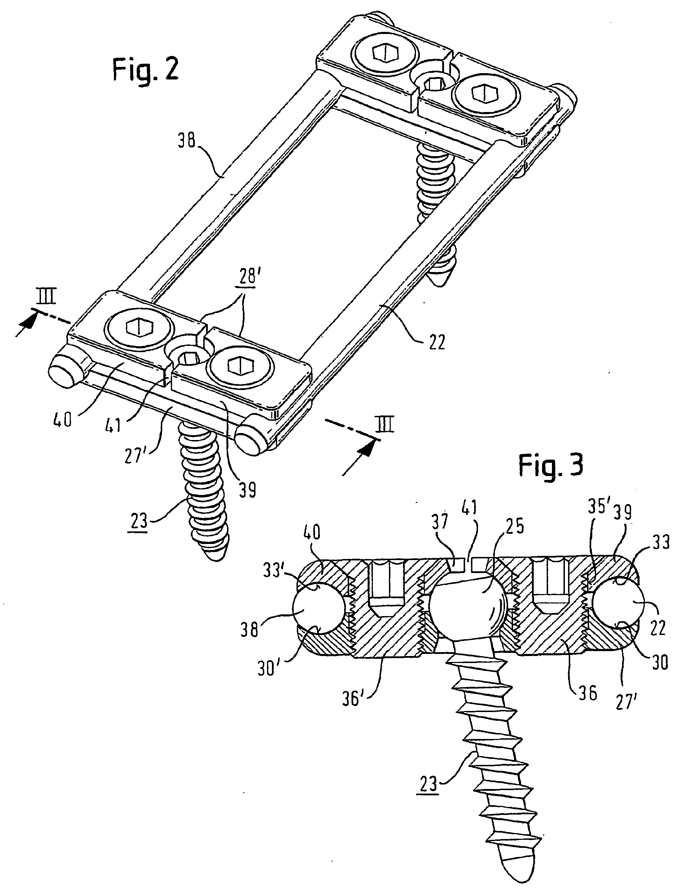

Die in Fig. 2 und 3 gezeigte Ausführungsform gehört nicht zur beanspruchten Erfindung und dient nur der technischen Erläuterung.

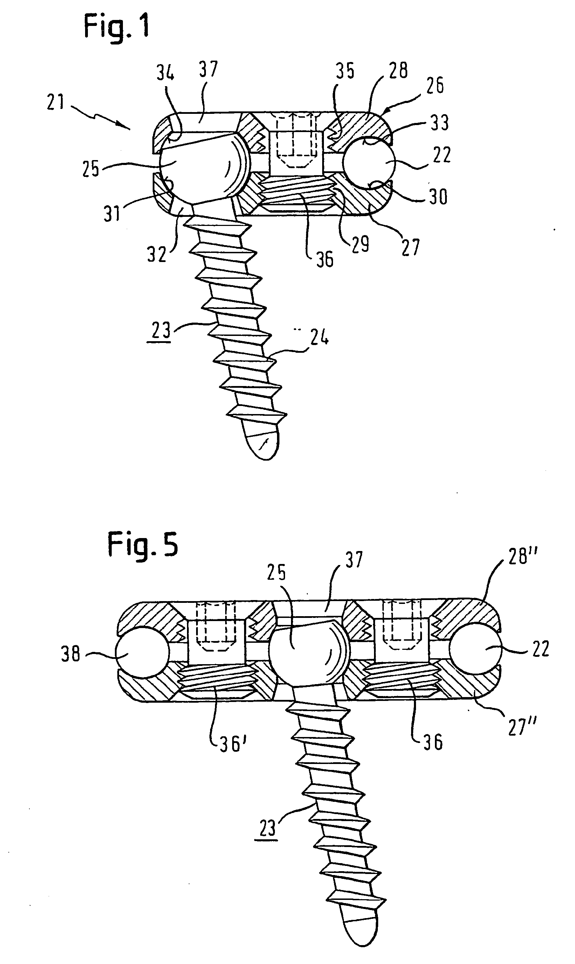

Zunächst wird mit Bezug auf Fig. 1 eine erste Ausführungsform der vorliegenden Erfindung beschrieben. Wie in Fig. 1 in einer seitlichen Schnittdarstellung gezeigt, weist eine Schraube 21 ein Schraubenelement 23 auf, welches ein Gewindeschaftteil 24 und einen sphärischen Kopf 25 aufweist, und eine Fassung 26, die ein Unterteil 27 und ein Oberteil 28 aufweist.

Unterteil 27 und Oberteil 28 sind identisch ausgebildet und werden symmetrisch zu einer durch die Mittellinie des Stabs und den Mittelpunkt des kugelförmigen Kopfs der Schraube definierten Ebene angeordnet.

Unterteil 27 und Oberteil 28 weisen je eine zentrale Bohrung auf, die mit einem Innengewinde 29, 35 versehen ist und auf der dem jeweils anderen Teil 27, 28 abgewandten Oberfläche eine Senkbohrung aufweist. Auf der einen Seite des zentralen Gewindes 29, 35 ist in einem Abstand von diesem eine zu dem jeweils anderen Teil 27, 28 hin zylindersegmentförmig ausgebildete Ausnehmung 30, 33 vorgesehen. Auf der dieser Ausnehmung gegenüberliegenden Seite des zentralen Gewindes 29, 35 weisen das Unterteil 127 und das Oberteil 28 auf der dem jeweils anderen Teil 27, 28 zugewandten Seite je eine kugelsegmentförmige Ausnehmung 31, 34 auf. Auf der dem anderen Teil 27, 28 abgewandten Oberfläche schließt sich koaxial zu der Ausnehmung 31, 34 eine vorzugsweise nach außen hin zunehmende kegelförmig ausgebildete Ausnehmung 32, 37 an.

Ferner ist eine Schraube 36 vorgesehen, die durch das Innengewinde 35 des Oberteils einführbar und in das Innengewinde 29 des Unterteiles in der in Fig. 1 gezeigten Weise einschraubbar ist. Die Schraube hat in ihrem durch das Oberteil 28 geführten Teil einen Durchmesser, der kleiner ist als der Innendurchmesser des Innengewindes 35 des Oberteils und weist in ihrem durch das Unterteil 27 geführten Teil ein mit dem Innengewinde 29 des Unterteils zusammenwirkendes Außengewinde auf.

Wie am besten aus Fig. 1 ersichtlich ist, weisen die beiden Ausnehmungen 30 und 33 den gleichen Radius auf, der dem Radius eines aufzunehmenden Stabes 22 entspricht. Ferner weisen die beiden Ausnehmungen 31 und 34 den gleichen Radius auf, der dem Radius des Kopfes 25 entspricht. Die Ausnehmungen sind jeweils um den gemeinsamen Mittelpunkt angeordnet und so bestimmt, dass Oberteil 28 und Unterteil 27 in der in Fig. 1 gezeigten Position, in der Stab und Kopf aufgenommen sind, parallel zueinander sind und einen Abstand voneinander besitzen.

Im Betrieb sind Unterteil 27 und Oberteil 28 durch Losdrehen der Schraube 36 ausreichend weit entfernt und werden dann um 90° gegeneinander verdreht. Es kann dann zunächst das Schraubenelement 23 durch die vorzugsweise kegelförmig ausgebildete Ausnehmung 32 des Unterteils 27 eingeführt und mit Hilfe eines entsprechenden Werkzeuges in ein zu fixierendes Teil eingeschraubt werden. Der Kopf weist zu diesem Zweck eine entsprechende Ausnehmung zum Eingreifen mit einem Schraubendreher auf. Anschließend wird der Stab 22 aufgenommen. Dann wird das Oberteil 28 um 90° in die in Fig. 1 gezeigte Position zurückgedreht, und es erfolgt dann ein Verbinden durch Anziehen der Schraube 36 in der in Fig. 1 gezeigten fixierten Position, in der sowohl die Schraube als auch der Stab von dem Kopf fest gehalten und somit miteinander verbunden sind. Die vorzugsweise kegelförmig ausgebildete Ausführung der Ausnehmung 32 des Unterteils 27 macht es möglich, Schraubenelement 23 und Fassung 26 polyaxial ausrichtbar miteinander zu verbinden. Durch die Ausnehmung 37 des Oberteils kann ein Schraubendreher eingeführt werden, so dass das Schraubenelement 23 auch noch betätigt werden kann, wenn sie bereits mit der Fassung 26 verbunden ist.

Eine mit solchen Schrauben gebildete Fixationseinrichtung umfasst wenigsten zwei solcher Schrauben 21 und einen damit zu verbindenden Stab 22, so dass zwei miteinander zu verbindende oder zu fixierende Teile über die so eingeschraubten Schrauben und den Stab miteinander in einer vorgewünschten Position gehalten werden.

Zur Fixation mehrerer Knochenteile gegeneinander kann es zum Erhöhen der Stabilität erwünscht sein, die Knochenschraube mit mehr als einem Stab zu verbinden. In Fig. 2 und 3 ist eine zweite Ausführungsform dargestellt, die in der Lage ist, zwei Stäbe mit dem Schraubenelement 23 zu verbinden.

Dazu ist die aus Unterteil 27' und Oberteil 28' bestehende Fassung auf eine dem ersten Stab 22 gegenüberliegende Seite verlängert zur Aufnahme eines zweiten Stabes 38. Diese Verlängerung kann so ausgeführt sein, dass Unterteil 27' und Oberteil 28' zu einer Ebene, die sich senkrecht zu der Ebene zwischen Oberteil und Unterteil und parallel zur Längsachse des Stabs 22 erstreckt und durch den Mittelpunkt der kugelsegmentförmigen Ausnehmungen (31, 34) verläuft, spiegelsymmetrisch ausgebildet sind, so dass auf der der Halterung für den Stab 22 gegenüberliegenden Seite eine gleichartige Halterung für einen zweiten Stab 38 vorgesehen ist. Zum Verklemmen ist wie die Schraube 36 zwischen dem Kopf 25 und dem Stab 22 eine entsprechende Schraube zwischen Kopf 25 und Stab 38 als Schraube 36' ausgebildet.

Unterteil 27' und Oberteil 28' sind in dieser Ausführungsform nicht identisch aufgebaut. Das Unterteil 27 weist beidseitig der kugelsegmentförmigen Aussparung 31 je eine Bohrung auf, die mit einem Innengewinde 29 versehen ist. Das Oberteil 28' weist koaxial zum Innengewinde 29 des Unterteils eine Bohrung 35' auf, die auf der dem Unterteil abgewandten Oberfläche eine Senkbohrung aufweist. Der Durchmesser der Bohrung 35' ist so gewählt, dass eine mit dem Innengewinde 29 zusammenwirkende Schraube durch die Bohrung hindurchführbar ist.

Wie aus den Fig. 2 und 3 ersichtlich ist, ist bei diesem Ausführungsbeispiel das Oberteil 28' so ausgebildet, dass die mit dem Kopf 25 und dem ersten Stab 22 zusammenwirkende erste Hälfte 39 und die mit dem Kopf 25 und dem zweiten Stab 38 zusammenwirkende, zweite Hälfte 40 durch einen Schlitz 41 voneinander getrennt sind, der sich quer zu einer Linie erstreckt, die durch die Mittelpunkte der Schrauben 36, 36' bestimmt ist.

Die anderen Merkmale entsprechen denen der ersten Ausführungsform und werden an dieser Stelle nicht wiederholt.

Mit dieser Ausführungsform können gleichzeitig zwei Stäbe 22 und 38 erfasst werden, wodurch eine wesentlich größere Stabilisierung der Halterung für entsprechende Anwendungszwecke erreicht wird.

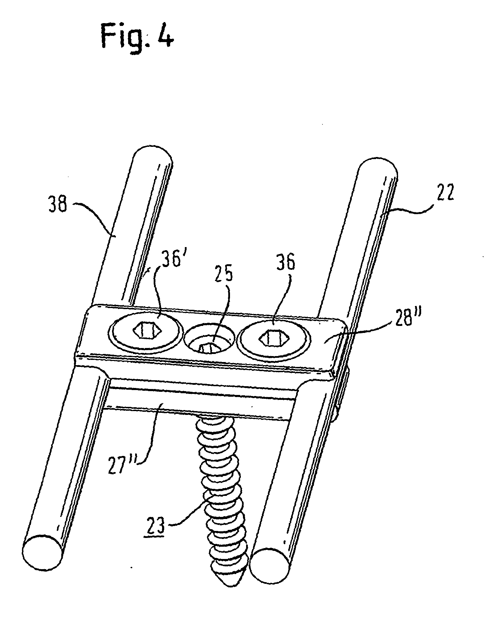

Eine in Fig. 4 dargestellte dritte Ausführungsform unterscheidet sich von der in Fig. 2 und 3 dargestellten zweiten Ausführungsform lediglich dadurch, dass das Oberteil 28" nicht geschlitzt ist.

Bei einer in Fig. 5 dargestellten vierten Ausführungsform sind auch bei der Ausführung zum Erfassen von zwei Stäben Oberteil und Unterteil identisch ausgebildet und werden symmetrisch zu einer durch die Mittellinie des Stabs 22 und den Mittelpunkt des kugelförmigen Kopfs 25 der Schraube definierten Ebene angeordnet.

Wie in der zweiten Ausführungsform sind Unterteil 27" und Oberteil 28'' in sich symmetrisch ausgebildet mit der kugelförmigen Aussparung 31, 34 in der Mitte. Wie in der ersten Ausführungsform weisen sowohl das Unterteil 27'' als auch das Oberteil 28'' beidseitig der kugelförmigen Aussparung je eine Bohrung auf, die mit einem Innengewinde 29, 35 versehen ist und auf der dem jeweils anderen Teil 27'', 28'' abgewandten Oberfläche eine Senkbohrung aufweist. Die beiden Schrauben 36, 36' haben in ihrem durch das Oberteil 28" geführten Teil einen Durchmesser, der kleiner ist als der Innendurchmesser des Innengewindes 35 des Oberteils und weisen in ihrem durch das Unterteil 27 geführten Teil ein mit dem Innengewinde 29 des Unterteils zusammenwirkendes Außengewinde auf.

Die anderen Merkmale entsprechen denen der zweiten Ausführungsform und werden an dieser Stelle nicht wiederholt.

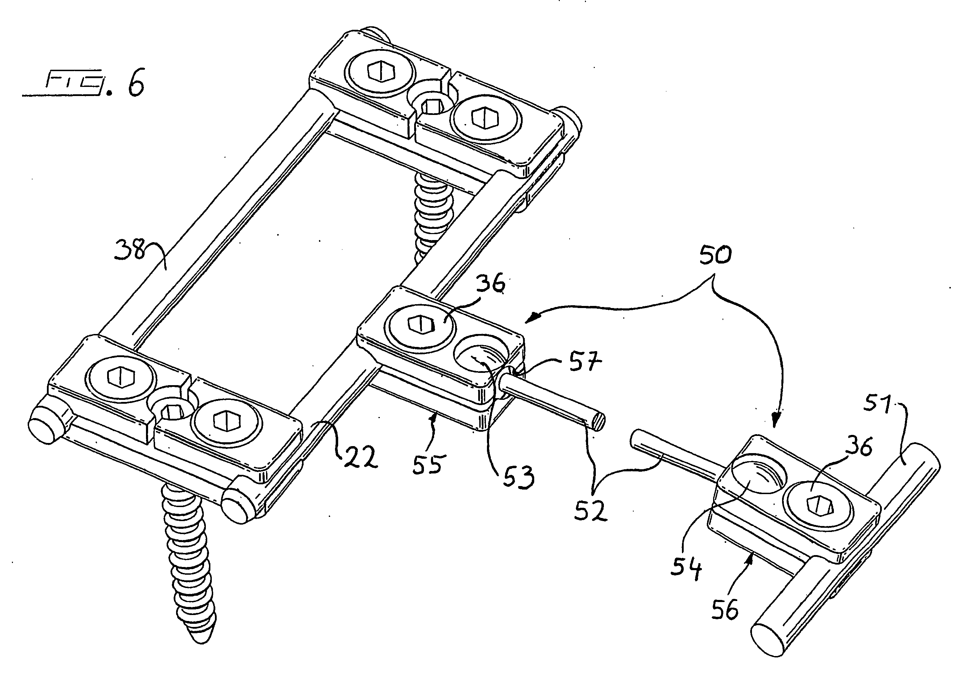

Eine in Fig. 6 dargestellte fünfte Ausführungsform stimmt bezüglich der zwei Stäbe 22, 38 und der diese verbindenden Fassung mit einer der in Fig. 2 bis 5 dargestellten Ausführungsformen vollständig überein. Fig. 6 zeigt zusätzlich eine Einrichtung 50 zum Verbinden eines weiteren Stabes 51 mit der Fixationseinrichtung. Die Einrichtung 50 weist einen als Schaft ausgebildeten Stab 52 auf, der gebrochen dargestellt ist und der an seinen beiden einander gegenüberliegenden Enden jeweils eine Kugel 53, 54 aufweist und an jedem seiner beiden Enden eine Fassung 55, 56, die.in dem gezeigten Ausführungsbeispiel identisch aufgebaut sind. Diese entsprechen in ihrem Aufbau der in Fig. 1 dargestellten Fassung 26 mit der Ausnahme, dass zusätzlich zu den vorzugsweise kegelförmigen Ausnehmungen 32 und 37 eine ebenfalls vorzugsweise nach außen hin zunehmende kegelförmig ausgebildete Ausnehmung 57 vorgesehen ist, deren Mittelachse durch den Mittelpunkt des für den Kopf 53, 54 gebildeten Lagers geht, das dem den Kopf 25 aufnehmenden Lager in Fig. 1 entspricht, und in dem gezeigten Ausführungsbeispiel in der Ebene zwischen Oberteil und Unterteil liegt und nicht nur durch die Mitte der Kugel 53, 54, sondern auch durch die Mittelachse der Schraube 36 läuft.

Die Abmessungen zwischen KugeI 53 bzw. 54 und dem Lager ist so bemessen, dass bei leichter Lockerung der Schraube 36 einerseits ein Verschieben am Stab 22 bzw. 51 möglich ist, andererseits um die Längsachse der vorzugsweise kegelförmig ausgebildeten Öffnung 57 ein um einen Kegel schwenkbares Bewegen des Schaftes 52 möglich ist, so dass auch nicht parallele Stäbe 22, 51 dieser Vorrichtung verbindbar sind. Nach dem verbinden wird die Schraube 36 festgezogen, wodurch die Fassung sowohl die Stäbe 22 bzw. 51 als auch die Kugelköpfe 53 bzw. 54 fest umfasst.

In dem in Fig. 6 gezeigten Ausführungsbeispiel ist die sphärische Ausnehmung 34 (Fig. 1) zur Oberseite des Oberteils 28 hin offen. Es ist aber genauso gut möglich, Oberteil und Unterteil ohne die Ausnehmungen 32, 37 auszubilden, so dass die sphärischen Ausnehmungen 31, 34 zur Außenseite hin geschlossen ausgebildet sind.

In Fig. 6 ist die Verbindungseinrichtung 50 im Zusammenhang mit einer der in Fig. 2 bis 5 dargestellten Ausführungsformen mit zwei Stäben 22, 38 gezeigt. Die Verbindungseinrichtung 50 kann aber ebenso gut gemeinsam mit der in Fig. 1 mit einem einzelnen Stab 22 dargestellten Ausführungsform verwendet werden.

Die Vorrichtung weist gegenüber dem eingangs erwähnten Stand der Technik die Vorteile auf, dass sie weniger Bauteile benötigt, und daher kostengünstiger herzustellen und einfacher zu handhaben. Da der Stab und die Schraube gleichzeitig fixiert werden, ist eine einfachere Justierung und somit insgesamt eine einfachere Handhabung gegeben. Ferner besteht der Vorteil einer extrem niedrigen Bauhöhe. Die Schraube kann mit vormontierter Fassung in den Knochen eingeschraubt werden, was die Handhabung weiter vereinfacht. Weiterhin ist es möglich, mehr als einen Stab mit einer Schraube zu verbinden. Die Vorrichtung kann auch dazu verwendet werden, Stäbe untereinander zu verbinden.

## Patentansprüche

1. Einrichtung zur Fixation von Knochen und insbesondere eines Beckens, mit wenigstens einer Schraube (21) für eine Fixationseinrichtung mit
einem Schraubenelement (23) mit einem Gewindeschaft (24) und einem kugelsegmentförmigen Kopf (25),
einer den Kopf (25) aufnehmenden Fassung (26) mit einem der Schaftseite zugewandten Unterteil (27; 27") und einem der Schaftseite abgewandten Oberteil (28; 28"), die zusammen einen Stab (22; 38) umfassen können, und
einem Schraubenelement (36; 36') zum Verbinden der beiden Teile (27; 27"; 28; 28") und gleichzeitigem Fixieren des Schraubenelements (23) und des Stabes (22; 38);
wobei das Oberteil (27; 27") und das Unterteil (28; 28") identisch ausgebildet und spiegelbildlich zueinander angeordnet sind, **dadurch gekennzeichnet, dass**
in dem Oberteil (27; 27") und dem Unterteil (28; 28") jeweils ein Gewinde (29, 35) zum Zusammenwirken mit dem die beiden Teile verbindenden Schraubenelement (36; 36') vorgesehen ist,
auf einer Seite des Gewindes (29, 35) einander zugewandte zylindersegmentförmige Ausnehmungen (30; 33) zum Aufnehmen des Stabes (22; 38) vorgesehen sind,
auf der gegenüberliegenden Seite des Gewindes (29, 35) einander zugewandte kugelsegmentförmige Ausnehmungen (31, 34) zur Aufnahme des Kugelkopfes (25) vorgesehen sind, und
zwischen den kugelsegmentförmigen Ausnehmungen (31, 34) und den einander abgewandten Deckflächen Ausnehmungen (32, 37) vorgesehen sind, zum Durchführen des Gewindeschaftes (24) einerseits und eines Werkzeuges zum Drehen des Schraubenelements (23) andererseits.

2. Fixationseinrichtung mit wenigsten zwei Schrauben (21) nach Anspruch 1 und einem von den Schrauben zu erfassenden Stab (22; 38).

3. Fixationseinrichtung nach Anspruch 1 wobei die den Kopf (25) aufnehmende Fassung (26) mit dem der Schaftseite zugewandten Unterteil (27") und dem der Schaftseite abgewandten Oberteil (28"), einen ersten Stab (22) und einen zweiten Stab (38) umfassen kann, und wobei
zwei Schraubenelemente (36, 36') zum Verbinden der beiden Teile (27", 28") und gleichzeitigem Fixieren des Schraubenelements (23) und der Stäbe (22, 38) vorgesehen sind.

4. Fixationseinrichtung nach Anspruch 3, **dadurch gekennzeichnet, dass** das Unterteil (27, 27") und das Oberteil (28, 28") in ihrer Mitte einander zugewandte kugelsegmentförmige Ausnehmungen (31, 34) zur Aufnahme des Kugelkopfes (25) aufweisen und um eine durch den Mittelpunkt der kugelförmigen Ausnehmung (34) sich erstreckenden Ebene spiegelsymmetrisch ausgebildet sind.

5. Fixationseinrichtung nach Anspruch 3 oder 4, **dadurch gekennzeichnet, dass** das Unterteil und das Oberteil auf den beiden Seiten zylindersegmentförmige Ausnehmungen (30, 33) zum Aufnehmen von zwei Stäben (22, 38) aufweisen.

6. Fixationseinrichtung nach einen der Ansprüche 3 bis 5 **dadurch gekennzeichnet, dass** das Unterteil (27; 27") und das Oberteil (28; 28") zwischen den kugelsegmentförmigen Ausnehmungen (31, 34) und den einander abgewandten Deckflächen Ausnehmungen (32, 37) aufweisen zum Durchführen des Gewindeschaftes (24) einerseits und eines Werkzeuges zum Drehen des Schraubenelements (23) andererseits.

7. Fixationseinrichtung mit wenigsten zwei Schrauben (21) nach einem der Ansprüche 3 bis 6 und zwei von den Schrauben zu erfassenden Stäben (22, 38).

## Claims

1. Device for the fixation of bones and in particular of a pelvis, with at least one screw (21) for a fixation device with
a screw element (23) with a threaded shaft (24) and a spherical segment-shaped head (25),
a mount (26) accommodating the head (25) with a lower part (27; 27'') facing the shaft side and an upper part (28, 28'') opposite to the shaft side, which can encompass a rod (22; 38), and
a screw element (36; 36') for connecting the two parts (27; 27"; 28; 28'') and simultaneously fixing of the screw element (23) and the rod (22; 38);
wherein the upper part (27; 27'') and the lower part (28; 28") are formed identically and arranged inversely with respect to each other, **characterized in that** in the upper part (27; 27'') and the lower part (28; 28'') a thread (29, 35; 35') for cooperating with the screw element (36; 36') connecting the two parts is provided,
on one side of the thread (29, 35) cylinder segment-shaped recesses (30; 33) facing each other for accommodating the rod (22; 38) are provided,
on the opposite side of the thread (29, 35, 35') spherical segment-shaped recesses (31, 34) facing each other for accommodating the spherical head (25) are provided, and
between the spherical segment-shaped recesses (31, 34) and the top faces which are opposite to each other, recesses (32, 37) are provided for passing through the threaded shaft (24) on one hand and a tool for rotating the screw element (23) on the other hand.

2. Fixation device with at least two screws (21) according to claim 1 and a rod (22; 38) to be captured by the screws.

3. Fixation device according to claim 1, wherein the mount (26') accommodating the head (25) which has a lower part (27, 27'') facing the shaft side and an upper part (28'') opposite to the shaft side, can encompass a first rod (22) and a second rod (38), and wherein
two screw elements (36, 36') are provided for connecting the two parts (27", 28") and simultaneously fixing of a screw element (23) and the rods (22, 38) are provided.

4. Fixation device according to claim 3, **characterized in that** the lower part (27, 27'') and the upper part (28, 28'') have in their center spherical segment-shaped recesses facing each other for the accommodation of the spherical head (25) and are inversely formed with respect to a plane extending through the center of the spherical-shaped recess (34).

5. Fixation device according to claim 3 or 4, wherein the lower part and the upper part have on both sides cylinder segment-shaped recesses (30, 33) for accommodating two rods (22, 38).

6. Fixation device according to one of claims 3 to 5,
wherein the lower part (27; 27") and the upper part (28; 28'') have recesses (32, 37) between the spherical segment-shaped recesses (31, 34) and the top faces opposite to each other, for passing through the threaded shaft (24) on the one hand and a tool for rotating the screw element (23) on the other hand.

7. Fixation device with at least two screws (21) according to one of claims 3 to 6 and two rods (22, 38) to be captured by the screws.

## Revendications

1. Dispositif pour la fixation osseuse, et en particulier d'un bassin, à au moins une vis (21) pour un moyen fixateur, comprenant
un élément en vis (23) à une tige filetée (24) et une tête en segment sphérique (25),
une douille de support (26) à recevoir ladite tête (25), qui comprend une partie inférieure (27 ; 27") se trouvant en face du côté de ladite tige ainsi qu'une partie supérieure (28 ; 28") opposée au côté de ladite tige, qui sont aptes à une action conjointe l'une avec l'autre en ceindrant une barre (22 ; 38), et
un élément en vis (36 ; 36') à relier lesdites deux parties (27 ; 27" ; 28 ; 28") et à fixer, en même temps, ledit élément en vis (23) et ladite barre (22 ; 38) ;
dans lequel ladite partie supérieure (27 ; 27") et ladite partie inférieure (28 ; 28") présentent une configuration identique et sont disposées en image symétrique l'une par rapport à l'autre,
**caractérisé en ce**
**qu'**un filetage respectif (29, 35 ; 35') est formé dans ladite partie supérieure (27 ; 27") et ladite partie inférieure (28 ; 28") pour une action conjointe avec ledit élément en vis (36 ; 36') reliant lesdites deux parties,
**que** des creux en segment de cylindre(30 ; 33), l'un en face de l'autre, sont formés d'un côté dudit filetage (29, 35, 35') à recevoir ladite barre (22 ; 38),
**que** des creux en segment sphérique (31, 34), l'un en face de l'autre, sont formés du côté opposé dudit filetage (29, 35, 35') à recevoir ladite tête sphérique (25), et
en ce que des creux (32, 37) sont formés entre lesdits creux en segment sphérique (31, 34) et les aires de recouvrement, l'une opposée à l'autre, afin d'y passer ladite tige filetée (24), d'un côté, et un outil à tourner ledit élément en vis (23), d'autre côté.

2. Dispositif de fixation à au moins deux vis (21) selon la revendication 1 et à une barre (22 ; 38) à saisir moyennant lesdites vis.

3. Dispositif de fixation selon la revendication 1, dans lequel
ladite douille de support (26') à recevoir ladite tête (25) est apte à une action conjointe avec ladite partie inférieure (27") en face du côté de ladite tige et avec ladite partie supérieure (28") opposée au côté de ladite tige, en ceindrant une première barre (22) et une deuxième barre (38), et dans lequel
deux éléments à vis (36, 36') sont utilisés afin de relier lesdits deux parties (27", 28") et fixer, en même temps, ledit élément en vis (23) et lesdites barres (22, 38).

4. Dispositif de fixation selon la revendication 3, **caractérisé en ce que** ladite partie inférieure (27, 27") et ladite partie supérieure (28, 28") comprennent, dans leurs centres, des creux en segment sphérique (31, 34), l'un en face de l'autre, à recevoir ladite tête sphérique (25), et sont configurés en image symétrique relativement à un plan s'étendant à travers le point central dudit creux sphérique (34).

5. Dispositif de fixation selon la revendication 3 ou 4, **caractérisé en ce que** ladite partie inférieure et ladite partie supérieure présentent des creux en segment de cylindre (30, 33) des deux côtés à recevoir deux barres (22, 38).

6. Dispositif de fixation selon une quelconque des revendications 3 à 5, **caractérisé en ce que** ladite partie inférieure (27 ; 27") et ladite partie supérieure (28 ; 28") présentent des creux (32, 37) entre lesdits creux en segment sphérique (31, 34) et les aires de recouvrement, opposées l'une à l'autre, afin d'y passer ladite tige filetée (24), d'un côté, et un outil à tourner ledit élément en vis (23), d'autre côté.

7. Dispositif de fixation à au moins deux vis (21) selon une quelconque des revendications 3 à 6 et à deux barres (22, 38) à saisir moyennant lesdites vis.
